# EUROPEAN PATENT SPECIFICATION

(11) **EP 3 644 821 B1**
(45) Date of publication and mention of the grant of the patent: **17.02.2021**
(21) Application number: 18732089.0
(22) Date of filing: 21.06.2018
(51) Int. Cl.: A61B 1/005, A61M 25/01

(54) **INVASIVE MEDICAL DEVICE WITH FLEXIBLE TIP**
INVASIVE MEDIZINISCHE VORRICHTUNG MIT FLEXIBLER SPITZE
DISPOSITIF MÉDICAL INVASIF À CONNEXION SOUPLE

(30) Priority: 28.06.2017 EP 17178361
(43) Date of publication of application: 06.05.2020
(73) Proprietor: Koninklijke Philips N.V., 5656 AG Eindhoven (NL)
(72) Inventor: DE JONG, Michiel, 5656 AE Eindhoven (NL); HAARTSEN, Jacob Roger, 5656 AE Eindhoven (NL); VAN DER BEEK, Maurice Hubertus Elisabeth, 5656 AE Eindhoven (NL); DE HAAS, Cornelis Gerardus Maria, 5656 AE Eindhoven (NL); VAN DEN BIJGAART, Adrianus Wilhelmus Dionisius M., 5656 AE Eindhoven (NL)
(74) Representative: Philips Intellectual Property & Standards
(86) International application number: PCT/EP2018/066684
(87) International publication number: WO 2019/002106

(56) References cited:
- JP-A- H05 184 525
- US-A- 4 846 573
- US-A- 5 357 979
- US-A1- 2011 230 758

## Description

### FIELD OF THE INVENTION

The present invention relates to an invasive medical device comprising a flexible elongate device portion attached to a handle, the flexible elongate device portion including a steerable flexible tip at a distal end of the flexible elongate device portion and a plurality of pull wires attached to the steerable flexible tip for steering the steerable flexible tip and extending into said handle.

The present invention further relates to an invasive medical device arrangement comprising such an invasive medical device.

The present invention yet further relates to an imaging system adapted to control such an invasive medical device arrangement.

### BACKGROUND OF THE INVENTION

Steerable invasive medical devices, e.g. steerable catheters or guidewires, are commonly used in medical procedures in order to steer the flexible tip of the invasive medical device towards a target area within a patient's body, e.g. a target location within the patient's heart, or to steer the flexible tip around an obstacle within the patient's body, such as a stenosis for example. Traditionally, such steering required the use of both hands of a medical practitioner, with the medical practitioner holding the handle of the invasive medical device in one hand whilst operating one or more rings or levers with the other hand to pull the pull wires attached to the steerable flexible tip in order to steer the tip, e.g. in a 3-D manner, such as up or down and left or right. This is rather complicated and requires a great deal of skill, in particular where simultaneous up/down and left/right adjustments of the steerable tip are required. Consequently, accurate steering of such an invasive medical device typically can only be achieved by experienced medical practitioners.

US 5,357,979 A discloses a flexible elongate device comprising a flexible elongate member having proximal and distal extremities. A shape-memory element is disposed in the flexible elongate member and is capable of assuming martensitic and austenitic states.

US 5,238,005 discloses a steerable flexible elongate device comprising a flexible elongate member having proximal and distal extremities and having a centrally disposed lumen extending into the distal extremity. The flexible elongate member has at least three additional lumens spaced apart circumferentially about the centrally disposed lumen and extending into the distal extremity. A stiffener element is disposed in the centrally disposed lumen and has proximal and distal extremities. Additional flexible elongate elements having a negative coefficient of expansion, e.g. elements made of nickel titanium alloy, are disposed in each of the three additional lumens and have proximal and distal extremities. The distal extremities of the stiffener element and the additional flexible elongate elements are secured to the distal extremity of the flexible elongate member. A control console including a joystick is provided to cause movement of the distal extremity through heating of the flexible elongate elements induced in accordance with the joystick position. To this end, a microprocessor coupled to the joystick translates the joystick orientation into a current that is supplied to the flexible elongate elements to heat the flexible elongate elements and cause them to shrink accordingly.

Such a steerable flexible elongate device may be operated more easily without the same levels of experience required for the manual operation of such a device using rings and levers. However, a disadvantage of this use of having flexible elongate elements having a negative coefficient of expansion extending into the distal extremity of such a steerable flexible elongate device is that the response time for the relaxation of a steered distal extremity after heating one or more of the flexible elongate elements can be inferior to arrangements including pull wires attached to the distal extremity, where release of the pull wires will yield instantaneous relaxation of the steerable distal extremity. Such rapid relaxation is desirable to limit the duration of medical procedures involving such steerable flexible elongate devices, for example to limit the duration of sedation or anaesthesia of the patient, to limit the period of stress or discomfort experienced by the patient. Also, the presence of flexible elongate elements in a flexible elongate device increases the risk of the blood or blood vessels of the patient becoming heated up during the procedure.

### SUMMARY OF THE INVENTION

The present invention seeks to provide an invasive medical device that has reduced relaxation times compared to the invasive medical devices of the art and avoids or at least reduces the risk of the blood or blood vessels of the patient becoming heated up during medical procedures with such an invasive medical device.

The present invention further seeks to provide an invasive medical device arrangement comprising such an invasive medical device.

The present invention yet further seeks to provide an imaging system adapted to control such an invasive medical device arrangement.

According to an aspect, there is provided an invasive medical device as defined in claim 1.

In accordance with the present invention, the steerable flexible tip is controlled by a wire arrangement comprising a plurality of pull wires extending through the flexible elongate device portion, with each pull wire being attached to an electrically conductive shape memory element located in the handle of the invasive medical device. This has the advantage of a more straightforward manufacturing of the flexible elongate device portion as no shape memory elements need to be incorporated within the flexible elongate device portion, whilst facilitating more rapid cooling of the shape memory elements in the handle owing to the large cross-sectional volume of the handle compared to the flexible elongate device portion, which facilitates a more rapid heat transfer from the electrically conductive shape memory elements towards their surrounding medium, as a larger volume of such a medium can be provided in the handle. In this manner, passive cooling of the electrically conductive shape memory elements may take place more rapidly compared to invasive medical devices in which the electrically conductive shape memory elements extend through the flexible elongate device portion and attach to the steerable flexible tip. Moreover, heat loss from the electrically conductive shape memory elements takes place outside the patient's body, thus avoiding the heating up of the patient's blood or blood vessels through such heat loss.

In a preferred embodiment, the invasive medical device further comprises a cooling arrangement in the handle arranged to cool the electrically conductive shape memory element to further reduce the relaxation time of the shape memory elements. For example, the cooling arrangement may comprise a heatsink arrangement including a plurality of channels, each of the shape memory element at least partially extending through one of said channels such that heat can be effectively transferred from the electrically conductive shape memory elements to the heatsink arrangement, thereby facilitating rapid cooling of the electrically conductive shape memory elements. Such a heatsink arrangement may be made of any suitable thermally conductive material. In an example embodiment, the heatsink arrangement comprises at least one metal or metal alloy heatsink, wherein each of said channels is lined with an electrically insulating material in order to prevent a short circuit between the heatsink arrangement and the electrically conductive shape memory elements.

Alternatively, the cooling arrangement may be arranged to generate a cooling fluid flow in thermal contact with the plurality of electrically conductive shape memory elements, such as an air flow or a liquid flow contacting the electrically conductive shape memory elements to achieve effective cooling of the electrically conductive shape memory elements. In yet another embodiment, the cooling arrangement implements solid-state active cooling, e.g. based on the Peltier effect.

The electrically conductive shape memory elements may be realized in any suitable manner. For example, each electrically conductive shape memory element may be a wire comprising a shape memory alloy although other shapes of shape memory element, e.g. strips or the like may also be used. Furthermore, any electrically conductive suitable shape memory material may be used for such electrically conductive shape memory elements.

Each electrically conductive shape memory element may be attached to a corresponding pull wire in any suitable manner. For example, each electrically conductive shape memory element may be attached to a pull wire through a securing member comprising a pair of screws for securing the electrically conductive shape memory element and the pull wire respectively in the securing member. This provides a particularly secure attachment of the electrically conductive shape memory element to the corresponding pull wire.

Each pull wire may extend through the flexible elongate device portion in any suitable manner. In a particularly suitable arrangement, each pull wire is housed in a lumen extending through the elongate device portion such that the pull wires can move freely through the flexible elongate device portion.

The invasive medical device according to embodiments of the present invention may be any suitable type of invasive medical device, such as for example a catheter or a guidewire. In some embodiments, the flexible elongate device portion is detachable from the handle. This for example is useful where the flexible elongate device portion is disposable, such that the handle may be reused by detaching it from the flexible elongate device portion and replacing it with a fresh flexible elongate device portion, e.g. for investigation or treatment of another patient. This therefore avoids that the entire invasive medical device has to be disposed after use, thus reducing cost.

A control unit may be provided with the invasive medical device according to any embodiment of the present invention to form an invasive medical device arrangement in accordance with a further aspect of the present invention. Such a control unit typically comprises a user interface such as a joystick coupled to a microcontroller and at least one current source for connecting to one of said current source connections of the invasive medical device, wherein the microcontroller is adapted to control the at least one current source to supply a current to the current source connection that is based on a user input provided with the user interface, e.g. an orientation of said joystick, such that the steerable flexible device tip can be controlled in a straightforward manner using the user interface.

To this end, the at least one current source may be a single source comprising a plurality of stages, with each of the stages being connected to one of the current source connections or alternatively the at least one current source comprises a plurality of current sources each connected to a different one of said current source connections.

The control unit may be a separate unit or alternatively may be integrated in the handle of the invasive medical device. The invasive medical device arrangement may be powered using a mains power supply or alternatively may include a battery coupled to the at least one current source in order to provide a battery-powered arrangement. Such a battery-powered arrangement optionally may also be powered using a mains power supply to yield a particularly flexible arrangement. In such an arrangement, the battery may be a rechargeable battery that may be charged in situ, e.g. by connecting the arrangement to the mains power supply or through wireless power transfer such as Qi, in which case the control unit may be made watertight for sterilisation purposes. Alternatively, the battery may be recharged by removal from the arrangement.

In a particularly advantageous arrangement, the control unit further comprises a communication module coupled to the microcontroller, and wherein the microcontroller is further adapted to control the at least one current source to supply a current to the current source connection that is based on a steering signal for steering the flexible tip received from an imaging system through the communication module. Consequently, the flexible tip of the invasive medical device may be steered automatically under control of the imaging system, without requiring manual intervention for the steering. In such a scenario, a medical practitioner may simply guide the invasive medical device into the patient or a mechanical guide arrangement may be provided to automate the guidance process as well.

According to yet another aspect, there is provided an imaging system as defined in claim 15. Such an imaging system may be used to automatically control the steering of the steerable flexible device tip of the invasive medical device as previously explained.

### BRIEF DESCRIPTION OF THE DRAWINGS

Embodiments of the invention are described in more detail and by way of non-limiting examples with reference to the accompanying drawings, wherein:
FIG. 1 schematically depicts an invasive medical device arrangement according to an embodiment;
FIG. 2 schematically depicts a cross-sectional view of an aspect of an invasive medical device arrangement according to an embodiment;
FIG. 3 schematically depicts a cross-sectional view of another aspect of an invasive medical device arrangement according to an embodiment;
FIG. 4 schematically depicts a cross-sectional view of such another aspect of an invasive medical device arrangement according to an alternative embodiment;
FIG. 5 schematically depicts an invasive medical device arrangement according to another embodiment;
FIG. 6 and 7 schematically depict an invasive medical device arrangement according to yet another embodiment;
FIG. 8 schematically depicts an invasive medical device arrangement according to yet another embodiment; and
FIG. 9 schematically depicts an example embodiment of an imaging system adapted to control an invasive medical device arrangement according to embodiments of the present invention.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

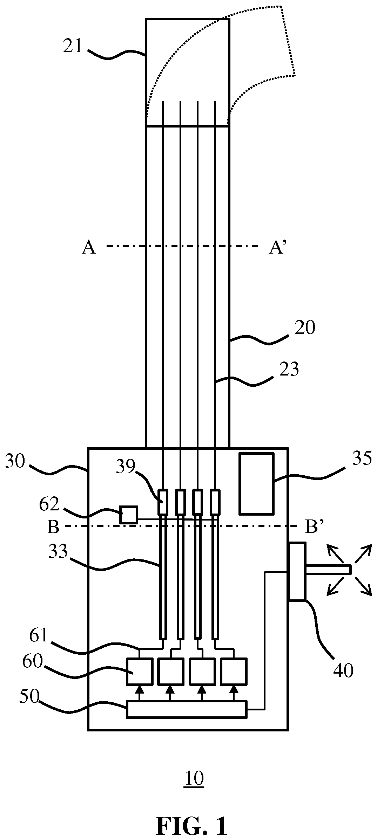

FIG. 1 schematically depicts an invasive medical device 10 according to an embodiment of the present invention. The invasive medical device 10 may be any type of medical device that may require insertion into a patient, such as any type of catheter or a guide wire. The invasive medical device 10 comprises a flexible elongate device portion or member 20 including a steerable flexible tip 21 at a distal end of the flexible elongate device portion 20. At its proximal end, the flexible elongate device portion 20 is attached to handle 30 used by a medical practitioner to hold the invasive medical device 10 whilst guiding the device within a patient. The flexible elongate device portion 20 may be made of any suitable flexible material, such as a flexible polymer, e.g. polyamide or the like and is typically intended to be at least partially inserted into the patient, e.g. into the patient's cardiovascular system, during a medical procedure in which the invasive medical device 10 is used. As will be explained in more detail below, the flexible elongate device portion 20 may be detachable from the handle 30 such that the flexible elongate device portion 20 can be replaced with a different flexible elongate device portion 20, e.g. a different type of flexible elongate device portion 20. Also, the flexible elongate device portion 20 may be disposable such that the handle 30 can be reused with a fresh (or resterilized) flexible elongate device portion 20.

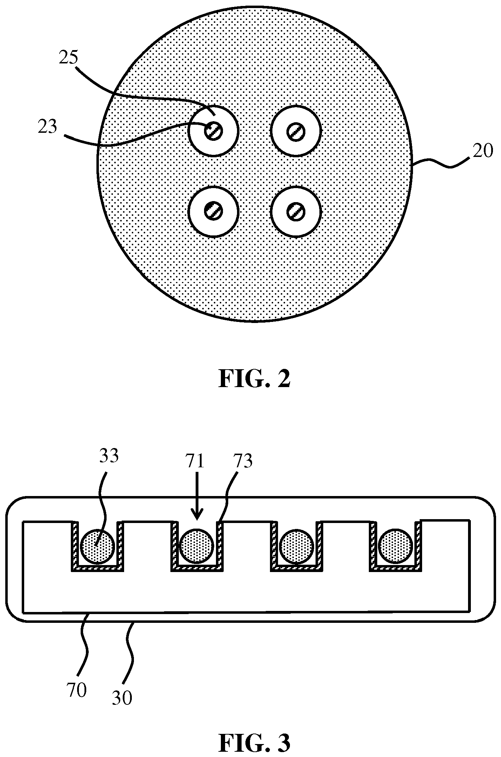

The steerable flexible tip 21, which may be steered or deformed as schematically indicated by the dashed deformation in FIG. 1, is attached to a plurality of pull wires 23 that extend through the flexible elongate device portion 20 and may be made of any suitable material, e.g. a polymer, metal or metal alloy. It is noted for the avoidance of doubt that any suitable number of pull wires 23 may be attached to the steerable flexible tip 21 of the flexible elongate device portion 20, and that four pull wires 23 are shown by way of non-limiting example only. The pull wires 23 may extend through the flexible elongate device portion 20 in any suitable manner. For example, each pull wire 23 may extend through a separate lumen 25 extending through the flexible elongate device portion 20 as schematically depicted in FIG. 2, which depicts a cross-sectional view of the flexible elongate device portion 20 along the line A-A' in FIG. 1.

Each pull wire 23 is attached to an electrically conductive shape memory element 33 contained within the handle 30 (from hereon simply referred to as shape memory element or SMA (shape memory alloy) 33). Each shape memory element 33 extends in a length direction between the pull wire 23 and a current source connection 61 that connects the shape memory element 33 to a current source 60, here located within the handle 30. Each shape memory element 33 has a negative coefficient of expansion in at least its length direction, such that upon supply of a current to the shape memory element 33 by its current source 60 through the current source connection 61, the shape memory element 33 shrinks in its length direction through heating, with the shape memory element 33 returning to its original dimension upon cooling of the shape memory element 33. Such heating may be achieved through the internal resistance of the SMA or through external heating. Because the shape memory element 33 is coupled to one of the pull wires 23, upon shrinkage the shape memory element 33 pulls its pull wire 23 towards the handle 30, thereby deforming, i.e. steering, the steerable flexible tip 21 of the flexible elongate device portion 20.

At this point it is noted that the current for heating the SMAs 33 may be delivered to the SMAs 33 in any suitable manner. For example, the SMAs 33 may form part of a closed conductive loop connected to the current source 60 through which the current is provided, or alternatively the SMAs 33 may be further connected to a current sink, e.g. ground, such that a current supplied to the SMAs 33 passes through the SMAs 33. This is schematically depicted in FIG. 1 by terminal 62, which may represent such a closed conductive loop or a current sink. A terminal 62 common to the SMAs 33 is shown by way of non-limiting example only, as it will be immediately understood by the skilled person that each SMA 33 may have its own terminal 62, e.g. in case of discrete closed conductive loops. This is not explicitly shown for the sake of clarity only. Many other suitable arrangements will be immediately apparent to the skilled person, and it should be understood that such arrangements are not shown for the sake of brevity only.

The shape memory elements 33 may be made of any suitable shape memory material, e.g. any suitable shape memory alloy having a negative coefficient of expansion. Such materials are well-known per se and are therefore not explained in further detail for the sake of brevity only. Each shape memory element 33 may have any suitable elongate shape, such as a wire, a strip or the like. A wire-shape comprising a shape memory alloy is particularly preferred.

Each shape memory element 33 may be attached to its corresponding pull wire 23 in any suitable manner, e.g. through spot welding or the like. In a particular embodiment, each shape memory element 33 is attached to its corresponding pull wire 23 through a securing member 39, which may comprise a conduit into which the shape memory element 33 and its corresponding pull wire 23 are inserted from opposite ends, which securing member 39 may further comprise a pair of screws arranged proximal to said ends and extending perpendicularly to the conduit such that by screwing down the screws the shape memory element 33 and its corresponding pull wire 23 can be secured in the securing member 39. This for example is particularly advantageous where the flexible elongate device portion 20 is disposable, such that the handle 30 may be reused as the pull wires 23 of the flexible elongate device portion 20 to be disposed can be easily released from the securing members 39 whilst the pull wires 23 of the replacement flexible elongate device portion 20 can be easily secured in the securing members 39 by loosening and tightening its screws.

The pull wires 23 may extend into the handle 30 where the pull wires 23 are secured to the shape memory elements 33 as previously explained although alternatively the shape memory elements 33 and/or the securing members 39 may extend beyond the perimeter of the handle 30, e.g. in order to facilitate replacement of a disposable flexible elongate device portion 20.

In the embodiment schematically depicted in FIG. 1, the invasive medical device 10 further comprises a user interface 40 attached to or otherwise integrated into the handle 30, which user interface 40 is used to steer the steerable flexible tip 21 of the flexible elongate device portion 20. The user interface 40 is communicatively coupled to a microcontroller 50 that translates the user interface signals into control signals for the respective current sources 60 in order to contract or shrink selected shape memory elements 33. For example, where the user interface 40 comprises a joystick, the microcontroller 50 translates the user interface signals into control signals for the respective current sources 60 in order to contract or shrink selected shape memory elements 33 corresponding to the position of the joystick 40 such that the flexible tip 21 is steered in accordance with this position. This may be achieved in any suitable manner, and as such joystick operation is well-known per se, this will not be explained in further detail for the sake of brevity only. Moreover, it should be understood that a joystick is just one of many typical examples of such a user interface 40, and that the user interface 40 may take any suitable alternative form, such as a trackball, a quadrant of push or touch buttons, and so on.

Although a plurality of discrete current sources 60 are shown, it should be understood that this is by way of non-limiting example only and that it is equally feasible to use a single current source 60 to control the respective shape memory elements 33, e.g. by having multiple stages each dedicated to one of the shape memory elements 33.

Although not specifically shown in FIG. 1, the microcontroller 50 may further be responsive to a feedback mechanism associated with each of the shape memory elements 33, in which the amount of shrinkage of the shape memory element 33 in response to the current supplied to the shape memory element 33 is quantified. This may be achieved in any suitable manner, for example with a temperature sensor thermally coupled to such a shape memory element 33, with the microcontroller 50 being responsive to the temperature sensor, or by the microcontroller 50 being arranged to measure the electrical resistance of the shape memory element 33, as this electrical resistance typically is a function of its degree of shrinkage. The microcontroller 50 may be arranged to operate the corresponding current source 60 in accordance with this feedback to ensure that the amount of desired shrinkage of the shape memory element 33 is accurately controlled and the flexible tip 21 is accurately steered as a consequence.

The control unit integrated in the handle 30, i.e. the control arrangement of the steerable flexible tip 21, may be powered through a mains power supply, in which case the invasive medical device 20 may include a power connection (not shown) such as a lead or the like for connecting the invasive medical device 10 to the mains power supply. Alternatively or additionally, the invasive medical device 20 may include a battery 35 that powers the invasive medical device 20 including the one or more current sources 60, the user interface 40 and the microcontroller 50. Such a battery 35 may be a rechargeable battery in some embodiments or a disposable battery in other embodiments. Where the battery 35 is a rechargeable battery, the battery 35 may be recharged in situ by connecting the invasive medical device 20 or the handle 30 to a mains power supply or by removing the battery 35 from the handle 30 in which the battery 35 may be integrated for recharging. Alternatively, the battery may be recharged in situ through wireless power transfer such as Qi, in which case the control unit may be made water tight, e.g. for sterilisation purposes. Where the control unit of the steerable flexible tip 21 is integrated in the handle 30 of the invasive medical device 10, the current source connections 61 connecting the shape memory elements 33 to one or more current sources 60 may be conductive tracks or the like that may be attached to the shape memory elements 33 in any suitable manner, e.g. through point soldering or welding, through use of a conductive glue or the use of any other suitable type of securing means.

As the handle 30 typically has a (much) larger cross-section than the insertable part of the invasive medical device 10, i.e. the flexible elongate device portion 20, upon resistive heating of one or more of the shape memory elements 33 with the one or more current sources 60 in response to a steering command provided with the user interface 40, the relaxation time of such a shape memory element 33 is typically reduced compared to an invasive medical device in which such a shape memory element extends through the flexible elongate device portion 20, i.e. the shape memory element 33 returns more quickly to its steady-state form due to more effective cooling of the shape memory element 33 in the handle 30. This is because the shape memory elements 33 may be further spaced apart in the handle 30 compared to in the flexible elongate device portion 20, such that the shape memory elements 33 may cool more quickly compared to a scenario in which the shape memory elements 33 need to be tightly packed within a flexible elongate device portion 20 due to the need to keep the cross-section of such a device portion 20 as small as possible because it needs to fit within the patient, e.g. needs to fit inside a blood vessel or organ of the patient.

Such effective cooling of the shape memory elements 33 may be achieved in a passive manner, e.g. through air within the handle 30, with the handle 30 comprising a plurality of openings, e.g. slits or the like, through which air can circulate such that hot air can be expelled from the handle 30 and be replaced with colder air, e.g. ambient air. Alternatively, the handle 30 may incorporate an active cooling arrangement to assist the cooling of the shape memory elements 33. For example, a fan or the like (not shown) may be incorporated in the handle 30 to force an air circulation through the handle 30 in order to assist the cooling of the shape memory elements 33.

In an example embodiment, a passive cooling arrangement comprises a heatsink 70 as shown in FIG. 3, which schematically depicts a cross-sectional view of the heatsink 70 along the dashed line B-B' in FIG. 1. The heatsink 70 typically comprises a plurality of channels 71 housing the shape memory elements 33. For example, each channel 71 may house a separate one of the shape memory elements 33. The shape memory elements 33 are housed within the heatsink 70 such that the shape memory elements 33 are thermally coupled to the heatsink 70. The heatsink 70 may have an open structure as schematically depicted in FIG. 3, in which the channels 71 are open in a length direction of the channels 73, or alternatively the heatsink 70 may envelop the shape memory elements 33 in which case the shape memory elements 33 extend through closed channels 71. The shape memory elements 33 typically comprise portions extending beyond the opposite boundaries of the heatsink 70 to facilitate the aforementioned dimensional changes of the shape memory elements 33 when controlling the steering of the steerable flexible tip 21.

The heatsink 70 may be made of any suitable thermally conductive material. Particularly suitable materials include metals, e.g. aluminium and metal alloys, e.g. steel. As such materials are electrically conductive, the channels 71 may be lined with an electrically insulating material, e.g. a coating 73 in the channels 71 to prevent a short-circuit between shape memory elements 33 through the electrically conductive heatsink 70. Such a coating 73 may be made of any suitable electrically insulating material. For example, the exposed surface of the heatsink 70 in the channels 71 may be oxidised to provide such an electrically insulating coating 73 or alternatively a coating 73 of an electrically insulating material may be deposited within the channels 71. Suitable electrically insulating materials include electrically insulating polymers and dielectric materials such as silicon oxide, silicon nitride and high-k dielectric materials.

The heatsink 70 may be combined with a further cooling arrangement, e.g. a fan or the like forcing air through the handle 30 as previously explained to accelerate the heat transfer from the heatsink 70 to its surroundings, e.g. ambient air.

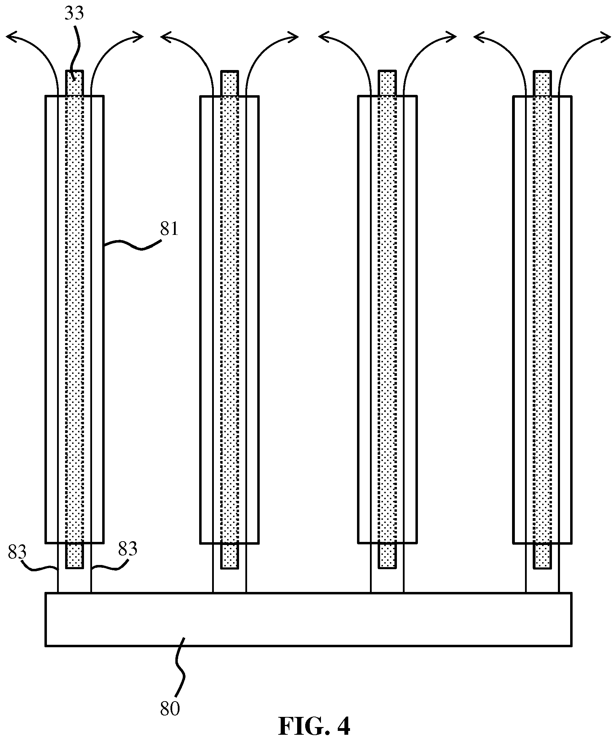

FIG. 4 schematically depicts an example embodiment of an active cooling arrangement 80 arranged within the handle 30 in which the cooling arrangement 80 generates a cooling fluid flow 83 in thermal contact with the shape memory elements 33. To this end, the shape memory elements 33 optionally are placed in conduits 81 through which the cooling fluid flow 83 is forced by the cooling arrangement 80. This for example may be desirable where the cooling fluid is a liquid in which case the conduits 81 of the cooling arrangement 80 may form part of a closed loop arrangement through which the liquid is pumped by a pump (not shown) of the cooling arrangement 80. Such a closed loop arrangement may be combined with a heatsink (not shown) in thermal contact with the closed loop arrangement to transfer the heat collected by the cooling liquid from the shape memory elements 33 to an ambient medium such as air. Alternatively, the conduits 81 may be omitted, e.g. where the cooling arrangement 80 generates an air flow around the shape memory elements 33 in order to cool the shape memory elements 33. Such an active cooling arrangement 80 may be preferable in application domains in which a particularly fast relaxation of the shape memory elements 33 is desired.

It should be understood that other examples of suitable cooling arrangements for the shape memory elements 33, e.g. thermoelectric cooling arrangements utilizing the Peltier effect, or any suitable combination of different types of cooling arrangements, e.g. combinations of passive and active cooling arrangements, may be used within the handle 30 to cool the shape memory elements 33.

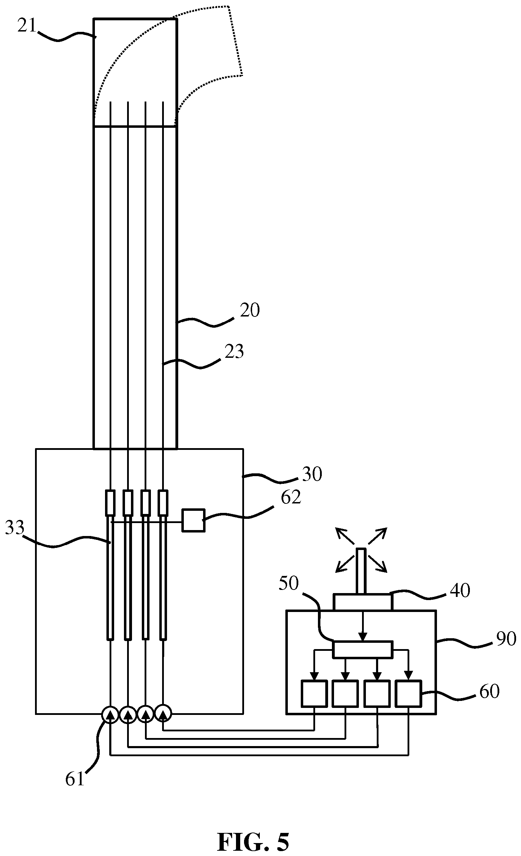

In FIG. 1, the control unit of the invasive medical device 10 is integrated in the handle 30. However, in an alternative embodiment as schematically depicted in FIG. 5, a separate control unit 90 may be provided comprising the user interface 40, e.g. a joystick or the like, and the microcontroller 50, and optionally further comprising the one or more current sources 60. In this embodiment, the current source connectors 61 of the shape memory elements 33 may include a plug or socket or the like through which the shape memory elements 33 may be connected to the one or more current sources 60 in the separate control unit 90. For example, each of the current sources or current source stages 60 may be connected to a plug, jack or the like that may be mated with a corresponding socket at a terminal end of a current source connector 61 exposed in the body of the handle 30 of the invasive medical device 10. Many other design variations will be immediately apparent to the skilled person and it should be understood that such design variations are intended to fall under the scope of the present invention.

As previously mentioned, the flexible elongate device portion or member 20 may be detachable from the handle 30, for example to facilitate interchanging of the flexible elongate device portion or member 20, i.e. in order to reuse the handle 30. This for example allows for the handle 30 to be reused with different types of flexible elongate device portions or members 20 and/or for the handle 30 to be reused with disposable flexible elongate device portions or members 20. This may be achieved using any suitable coupling mechanism between the flexible elongate device portion or member 20 and the handle 30 such as the previously mentioned securing members 39.

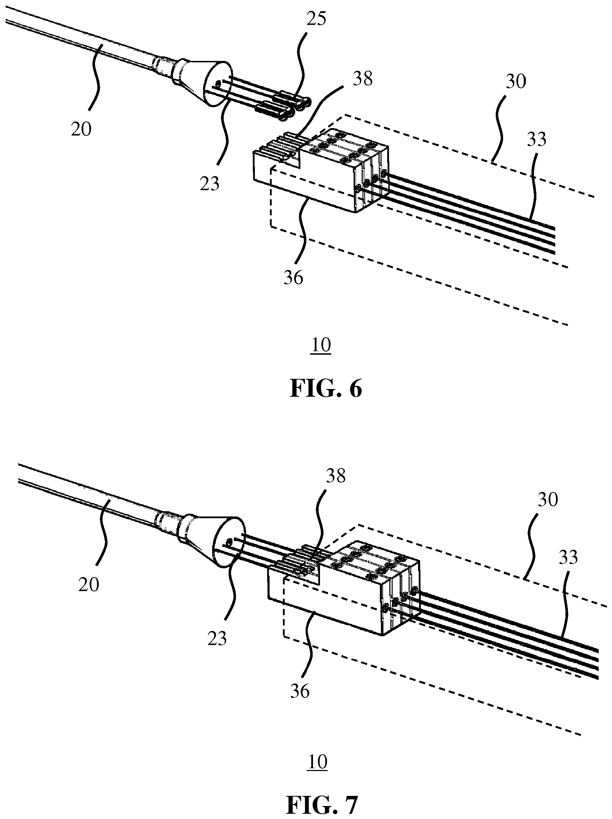

FIG. 6 and 7 schematically depict another example embodiment of such a securing arrangement in which the handle 30 comprises a connection block 36 for engaging with such an interchangeable flexible elongate device portion or member 20. Each electrically conductive shape memory element 33 within the handle 30 is mechanically coupled to a channel or groove 38 in the connection block 36, which acts as a securing member for the connectors 25 at the terminals of the pull wires 23 in the flexible elongate device portion or member 20 proximal to the handle 30, and which may be external to the handle 30 for easy access. The connectors 25 and the channels or grooves 38 may have a shape such as a mushroom shape or any other suitable shape such that the connectors 25 can be easily secured in the channels or grooves 38, as schematically depicted in FIG. 7 . The channels or grooves 38 typically are movably mounted in the connection block 36 such that dimensional changes in the electrically conductive shape memory elements 33 are translated to the pull wires 23 to which these electrically conductive shape memory element 33 are attached. This arrangement allows for a particularly easy connection and disconnection of a flexible elongate device portion or member 20 to the handle 30 by positioning or removal of the connectors 25 from the channels or grooves 38 as will be appreciated by the skilled person.

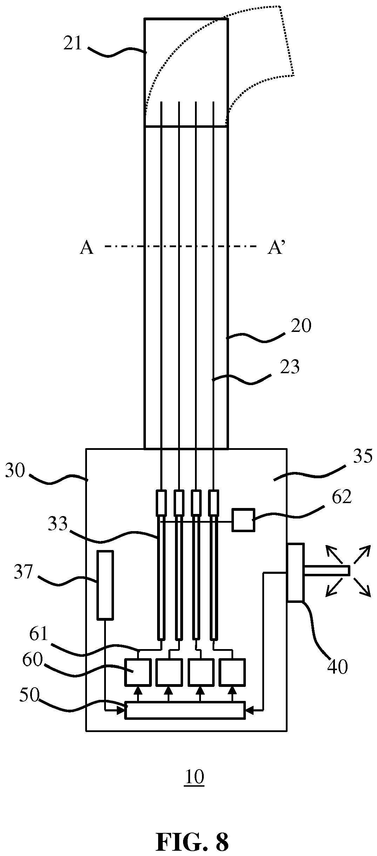

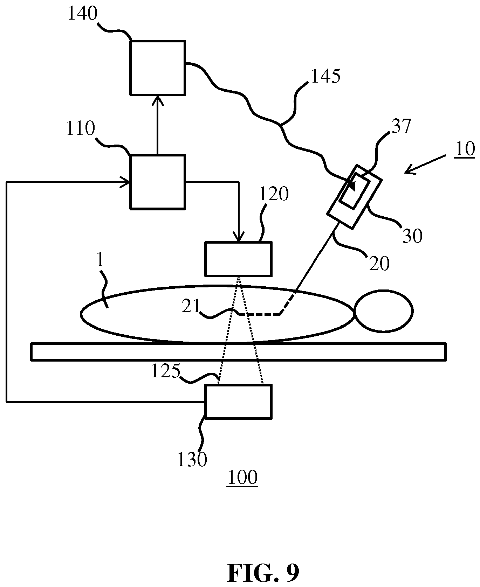

FIG. 8 schematically depicts another embodiment of the invasive medical device 10 of the present invention, in which the control unit, here integrated in the handle 30 by way of non-limiting example only, further comprises a communication module 37 arranged to communicate with a imaging system, such as the imaging system 100 as schematically depicted in FIG. 9. The communication module 37 may be arranged to communicate with such an imaging system in any suitable manner, e.g. in a wireless fashion using any suitable wireless communication protocol such as Bluetooth or Wi-Fi by way of non-limiting example or alternatively the communication module 37 may be arranged to communicate with such an imaging system in a wired fashion, in which case the communication module 37 may be (temporarily) coupled to the imaging system 100 through a cable or the like as is well-known per se.

The communication module 37 is coupled to the microcontroller 50, with the microcontroller 50 being adapted to receive a steering command 145 for the steerable flexible tip 21 of the flexible elongate device portion 20 from the imaging system 100 through the communication module 37 and to translate this steering command into one or more control signals for the one or more current sources 60 in order to force dimensional change in one or more of the shape memory elements 33 to automatically steer the steerable flexible tip 21 in accordance with the steering command. If the flexible tip 21 is steered in this manner, a medical professional operating the invasive medical device 10 may only be required to guide the invasive medical device 10 through the patient 1 or alternatively such guidance is also automated, e.g. under control of the imaging system 100.

The imaging system 100 may generate the steering command 145 based on an evaluation of one or more images generated with the imaging system 100 of a body portion of the patient 1 in which at least the steerable flexible tip 21 of the flexible elongate device portion 20 of the invasive medical device 10 is located. To this end, the imaging system 100 may comprise an electromagnetic radiation generator 120 under control of a processor arrangement 110, which electromagnetic radiation generator 120 generates electromagnetic radiation 125 through the body portion of the patient 1 containing the steerable flexible tip 21 in order to image the orientation of the steerable flexible tip 21 within the body portion of the patient 1. The imaging system 100 typically further comprises an electromagnetic radiation collector 130 for collecting the modified electromagnetic radiation 125 as it has passed through the body portion of the patient 1. For example, the electromagnetic radiation generator 120 may generate x-ray radiation, a magnetic field, ultrasound waves or the like that are collected by the electromagnetic radiation collector 130 and fed to the processor arrangement 110, where the collected electromagnetic radiation is processed and converted into one or more images of the body portion of the patient 1.

As will be readily understood by the skilled person, the electromagnetic radiation collector 130 may be a separate entity to the electromagnetic radiation generator 120, for example in the case of an x-ray imaging system, a CT imaging system, an MRI system or the like, or may be integrated or form part of the electromagnetic radiation generator 120, for example in the case of an ultrasound imaging system comprising a transducer array adapted to generate ultrasound waves and collect echoes of such ultrasound waves as is well-known per se.

The electromagnetic radiation collector 130 is typically adapted to convert the collected electromagnetic radiation into one or more electrical signals that are fed to the processor arrangement 110, which typically includes an image processor adapted to process the one or more electrical signals and convert those signals into an image or a plurality of images of the body portion of the patient 1 imaged with the imaging system 100. The image processor of the processor arrangement 110 may be adapted to identify the steerable flexible tip 21 and its orientation within the body portion of the patient 1 into one or more images of this body portion. This for example may be achieved using well-known object recognition algorithms.

For example, the steerable flexible device tip 21 and/or the flexible elongate device portion 20 may include a plurality of spatially distributed markers in accordance with a defined spatial distribution that can be recognized within the one or more images, such that the orientation of the steerable flexible device tip 21 within the body portion of the patient 1 may be derived from the recognized markers and the defined spatial distribution of these markers. Many other ways of recognising the orientation of the steerable flexible device tip 21 within the patient's body will be immediately apparent to the skilled person, as such recognition techniques are well-known per se and are therefore not explained in further detail for the sake of brevity only.

The image processor, or any other processor of the processor arrangement 110 typically is arranged to compare the actual orientation of the steerable flexible device tip 21 including the actual shape of the steerable flexible device tip 21 as derived from the one or more generated images against a desired orientation of the steerable flexible device tip 21. Such a desired orientation may be specified by a controller, e.g. a medical practitioner, of the imaging system 100, e.g. through a user interface of the imaging system 100 such as a user console the like, or may be automatically derived by the imaging system 100 from the one or more generated images, for example by the recognition of an object in the vicinity of the steerable flexible device tip 21 to which or around which the steerable flexible device tip 21 should be steered.

The processor arrangement 110 generates a steering command 145 for the steerable flexible device tip 21 based on the determined difference between the actual orientation of the steerable flexible device tip 21 and its desired orientation within the patient's body. The processor arrangement 110 typically is coupled to a communication module 140 of the imaging system 100, which communication module 140 is arranged to communicate the steering command 145 to the communication module 37 of the invasive medical device 10, e.g. in a wireless or wired fashion as previously explained, such that the microcontroller 50 can generate the control signals for the one or more shape memory elements 33 in the handle 30 of the invasive medical device 10 in accordance with the received steering command 145 in order to steer the device tip 21 towards its desired orientation within the patient's body.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An invasive medical device (10) comprising:
a flexible elongate device portion (20) attached to a handle (30), the elongate device portion including a steerable flexible tip (21) at a distal end of the elongate device portion and a plurality of pull wires (23) for steering the steerable flexible tip attached to said steerable flexible tip; and
a plurality of electrically conductive shape memory elements (33) entirely contained within said handle, each of the shape memory elements attached to and extending in a length direction between one of said pull wires and a current source connection (61), and wherein each of the shape memory elements having a negative coefficient of expansion in said length direction is configured to allow flow of electrical current between the current source connection and a terminal.

2. The invasive medical device (10) of claim 1, further comprising a cooling arrangement (70, 80) in the handle (30) arranged to cool the plurality of electrically conductive shape memory elements (33).

3. The invasive medical device (10) of claim 2, wherein the cooling arrangement comprises a heatsink arrangement (70) including a plurality of channels (71), each of the electrically conductive shape memory elements (33) at least partially extending through one of said channels.

4. The invasive medical device (10) of claim 3, wherein the heatsink arrangement (70) comprises at least one metal or metal alloy heatsink, and wherein each of said channels (71) is lined with an electrically insulating material (73).

5. The invasive medical device (10) of any of claims 2-4, wherein the cooling arrangement (80) is arranged to generate a cooling fluid flow (83) in thermal contact with the plurality of electrically conductive shape memory elements (33).

6. The invasive medical device (10) of any of claims 1-5, wherein each electrically conductive shape memory element (33) is a wire comprising a shape memory alloy.

7. The invasive medical device (10) of any of claims 1-6, wherein each electrically conductive shape memory element (33) is attached to a pull wire (23) through a securing member (39) comprising a pair of screws for securing the electrically conductive shape memory element and the pull wire respectively in the securing member.

8. The invasive medical device (10) of any of claims 1-7, wherein each pull wire (23) is housed in a lumen (25) extending through at least a part of the flexible elongate device portion (20).

9. The invasive medical device (10) of any of claims 1-8, wherein the invasive medical device is a catheter or a guidewire, optionally wherein the flexible elongate device portion (20) is detachable from the handle (30).

10. An invasive medical device arrangement comprising the invasive medical device (10) of any of claims 1-9 and a control unit (90), the control unit comprising a user interface (40) coupled to a microcontroller (50) and at least one current source (60) for connecting to one of said current source connections (61) of the invasive medical device, wherein the microcontroller is adapted to control the at least one current source to supply a current to the current source connection that is based on a user input provided with said user interface (40).

11. The invasive medical device arrangement of claim 10, wherein the at least one current source (60) comprises a plurality of current sources each connected to a different one of said current source connections (61).

12. The invasive medical device arrangement of claim 10 or 11, wherein the control unit (90) is integrated in the handle (30) of the invasive medical device (10).

13. The invasive medical device arrangement of claim 12, further comprising a battery (35) coupled to the at least one current source (60).

14. The invasive medical device arrangement of any of claims 10-13, wherein the control unit (90) further comprises a communication module (37) coupled to the microcontroller (50), and wherein the microcontroller is further adapted to control the at least one current source (60) to supply a current to the current source connection (61) that is based on a steering signal (145) for steering the steerable flexible tip (21) received from an imaging system (100) through the communication module.

15. An imaging system (100) comprising:
the invasive medical device arrangement of claim 14;
an image generator including an electromagnetic radiator generator (120) and an electromagnetic radiation collector (130) for generating an image of a portion of a patient's body (1), said portion comprising at least the steerable flexible tip (21) of the invasive medical device (10);
an image processor (110) coupled to the electromagnetic radiation collector and adapted to:
process said image to determine an actual orientation of the steerable flexible tip within the portion of the patient's body;
determine a difference between said actual orientation and a desired orientation of the steerable flexible tip;
generate a steering signal (145) for the steerable flexible tip based on said difference; and
provide the steering signal to the communication module (37) of the invasive medical device arrangement.

## Patentansprüche

1. Ein invasives medizinisches Gerät (10), das Folgendes umfasst:
einen flexiblen länglichen Geräteabschnitt (20), der an einem Griff (30) angebracht ist, wobei der längliche Geräteabschnitt eine lenkbare flexible Spitze (21) am distalen Ende des länglichen Geräteabschnitts sowie eine Vielzahl von Zugdrähten (23) an der lenkbaren flexiblen Spitze zum Lenken der lenkbaren flexiblen Spitze umfasst; und
mehrere elektrisch leitende Formgedächtniselemente (33), die vollständig in den Griff integriert sind, wobei die einzelnen Formgedächtniselemente zwischen einem der Zugdrähte und dem Stromanschluss (61) angebracht sind und sich in Längsrichtung zwischen diesen erstrecken, und
wobei die einzelnen Formgedächtniselemente über einen negativen Ausdehnungskoeffizienten in Längsrichtung verfügen und den Fluss des elektrischen Stroms zwischen dem Stromanschluss und einer Klemme ermöglichen.

2. Das invasive medizinische Gerät (10) gemäß Anspruch 1, wobei dieses zudem eine Kühlvorrichtung (70, 80) im Griff (30) umfasst, die die elektrisch leitenden Formgedächtniselemente (33) kühlt.

3. Das invasive medizinische Gerät (10) gemäß Anspruch 2, wobei die Kühlvorrichtung eine Kühlkörperanordnung (70) mit mehreren Kanälen (71) umfasst, und wobei sich die einzelnen elektrisch leitenden Formgedächtniselemente (33) zumindest teilweise durch einen der Kanäle erstrecken.

4. Das invasive medizinische Gerät (10) gemäß Anspruch 3, wobei die Kühlkörperanordnung (70) mindestens einen Kühlkörper aus Metall oder aus einer Metalllegierung umfasst, und wobei die einzelnen Kanäle (71) mit einem elektrisch isolierenden Material (73) ausgekleidet ist.

5. Das invasive medizinische Gerät (10) gemäß einem der Ansprüche 2 bis 4, wobei die Kühlanordnung (80) einen in thermischem Kontakt mit den elektrisch leitenden Formgedächtniselementen (33) stehenden Kühlflüssigkeitsstrom (83) erzeugt.

6. Das invasive medizinische Gerät (10) gemäß einem der Ansprüche 1 bis 5, wobei es sich bei den einzelnen elektrisch leitenden Formgedächtniselementen (33) um einen Draht mit einer Formgedächtnislegierung handelt.

7. Das invasive medizinische Gerät (10) gemäß einem der Ansprüche 1 bis 6, wobei die einzelnen elektrisch leitenden Formgedächtniselemente (33) mit einem Befestigungselement (39) an einem Zugdraht (23) befestigt sind, das ein Schraubenpaar zum Befestigen des elektrisch leitenden Formgedächtniselements bzw. des Zugdrahts am Befestigungselement umfasst.

8. Das invasive medizinische Gerät (10) gemäß einem der Ansprüche 1 bis 7, wobei sich die einzelnen Zugdrähte (23) in einem Lumen (25) befinden, das sich durch mindestens einen Teil des flexiblen länglichen Geräteabschnitts (20) erstreckt.

9. Das invasive medizinische Gerät (10) gemäß einem der Ansprüche 1 bis 8, wobei es sich beim invasiven medizinischen Gerät um einen Katheter oder einen Führungsdraht handelt, und wobei der flexible längliche Geräteabschnitt (20) optional vom Griff (30) abgenommen werden kann.

10. Eine invasive medizinische Geräteanordnung, die das invasive medizinische Gerät (10) gemäß einem der Ansprüche 1 bis 9 sowie eine Steuerungseinheit (90) umfasst, wobei die Steuerungseinheit eine mit einem Mikrocontroller (50) verbundene Benutzerschnittstelle (40) sowie mindestens eine Stromquelle (60) zum Anschließen an einem der Stromanschlüsse (61) des invasiven medizinischen Geräts umfasst,
wobei der Mikrocontroller die mindestens eine Stromquelle steuert, um den Stromanschluss mit Strom zu versorgen. Dies beruht auf einer Benutzereingabe mithilfe der Benutzerschnittstelle (40).

11. Die invasive medizinische Geräteanordnung gemäß Anspruch 10, wobei die mindestens eine Stromquelle (60) mehrere Stromquellen umfasst, die jeweils an einem anderen der Stromanschlüsse (61) angeschlossen wird.

12. Die invasive medizinische Geräteanordnung gemäß Anspruch 10 oder 11, wobei die Steuerungseinheit (90) in den Griff (30) des invasiven medizinischen Geräts (10) integriert ist.

13. Die invasive medizinische Geräteanordnung gemäß Anspruch 12, die zudem über einen an der mindestens einen Stromquelle (60) angeschlossenen Akku (35) verfügt.

14. Die invasive medizinische Geräteanordnung gemäß einem der Ansprüche 10 bis 13, wobei die Steuerungseinheit (90) zudem ein Kommunikationsmodul (37) umfasst, das mit dem Mikrocontroller (50) verbunden ist, und wobei der Mikrocontroller zudem die mindestens eine Stromquelle (60) steuert, um den Stromanschluss (61) mit Strom zu versorgen. Dies beruht auf einem Lenksignal (145) zum Lenken der lenkbaren flexiblen Spitze (21), das vom Kommunikationsmodul von einem Bildgebungssystem (100) abgerufen wird.

15. Ein Bildgebungssystem (100), das Folgendes umfasst:
die invasive medizinische Geräteanordnung gemäß Anspruch 14;
einen Bildgenerator mit einem elektromagnetischen Strahlungsgenerator (120) und
einen elektromagnetischen Strahlungskollektor (130) zum Erzeugen eines Bilds eines Teils des Körpers (1) eines Patienten, wobei der Teil mindestens die lenkbare flexible Spitze (21) des invasiven medizinischen Geräts (10) umfasst;
einen Bildprozessor (110), der mit dem elektromagnetischen Strahlungskollektor verbunden ist und folgende Schritte durchführt:
Verarbeiten des Bilds, um die tatsächliche Ausrichtung der lenkbaren flexiblen Spitze innerhalb des Körpers des Patienten zu ermitteln;
Ermitteln des Unterschieds zwischen der tatsächlichen und der gewünschten Ausrichtung der lenkbaren flexiblen Spitze;
Erzeugen eines Lenksignals (145) für die lenkbare flexible Spitze anhand des Unterschieds; und
Bereitstellen des Lenksignals für das Kommunikationsmodul (37) der invasiven medizinischen Geräteanordnung.

## Revendications

1. Dispositif médical (10) invasif comprenant :
une partie de dispositif (20) allongée flexible fixée à une poignée (30), la partie de dispositif allongée comprenant une pointe (21) flexible orientable à une extrémité distale de la partie de dispositif allongée et une pluralité de fils de traction (23) pour diriger la pointe orientable flexible attachées à ladite pointe flexible orientable ; et
une pluralité d'éléments à mémoire de forme (33) électriquement conducteurs entièrement contenus dans ladite poignée, chacun des éléments à mémoire de forme étant attaché à et s'étendant dans une direction de longueur entre l'un desdits fils de traction et une connexion de source de courant (61), et dans lequel chacun des éléments à mémoire de forme présentant un coefficient d'expansion négatif dans ladite direction de longueur est conçu pour permettre la circulation du courant électrique entre la connexion de source de courant et une borne.

2. Dispositif médical (10) invasif selon la revendication 1, comprenant en outre un agencement de refroidissement (70, 80) dans la poignée (30) conçu pour refroidir la pluralité d'éléments à mémoire de forme (33) électriquement conducteurs.

3. Dispositif médical (10) invasif selon la revendication 2, dans lequel l'agencement de refroidissement comprend un agencement de dissipateur thermique (70) comprenant une pluralité de canaux (71), chacun des éléments à mémoire de forme (33) électriquement conducteurs s'étendant au moins partiellement à travers l'un desdits canaux.

4. Dispositif médical (10) invasif selon la revendication 3,dans lequel l'agencement de dissipateur de chaleur (70) comprend au moins un dissipateur de chaleur en métal ou en alliage métallique, et dans lequel chacun desdits canaux (71) est revêtu d'une matière électriquement isolante (73).

5. Dispositif médical (10) invasif selon l'une quelconque des revendications 2 à 4, dans lequel l'agencement de refroidissement (80) est conçu pour générer un écoulement de fluide de refroidissement (83) en contact thermique avec la pluralité d'éléments à mémoire de forme (33) électriquement conducteurs.

6. Dispositif médical (10) invasif selon l'une quelconque des revendications 1 à 5, dans lequel chaque élément à mémoire de forme (33) électriquement conducteur est un fil comprenant un alliage à mémoire de forme.

7. Dispositif médical (10) invasif selon l'une quelconque des revendications 1 à 6, dans lequel chaque élément à mémoire de forme (33) électriquement conducteur est fixé à un fil de traction (23) à travers un élément de fixation (39) comprenant une paire de vis pour fixer respectivement l'élément à mémoire de forme électriquement conducteur et le fil de traction dans l'élément de fixation.

8. Dispositif médical (10) invasif selon l'une quelconque des revendications 1 à 7, dans lequel chaque fil de traction (23) est logé dans une lumière (25) s'étendant à travers au moins une partie de la partie de dispositif allongée (20) flexible.

9. Dispositif médical (10) invasif selon l'une quelconque des revendications 1 à 8, dans lequel le dispositif médical invasif est un cathéter ou un fil guide, éventuellement dans lequel la partie de dispositif allongée (20) flexible est détachable de la poignée (30).

10. Agencement de dispositif médical invasif comprenant le dispositif médical (10) invasif selon l'une quelconque des revendications 1 à 9 et une unité de commande (90), l'unité de commande comprenant une interface utilisateur (40) couplée à un microcontrôleur (50) et au moins une source de courant (60) pour être connectée à l'une desdites connexions de source de courant (61) du dispositif médical invasif, dans lequel le microcontrôleur est conçu pour commander l'au moins une source de courant pour fournir un courant à la connexion de source de courant, laquelle est basé sur une entrée utilisateur fournie à l'aide de ladite interface utilisateur (40).

11. Agencement de dispositif médical invasif selon la revendication 10, dans lequel l'au moins une source de courant (60) comprend une pluralité de sources de courant connectées chacune à une connexion différente desdites connexions de source de courant (61).

12. Agencement de dispositif médical invasif selon la revendication 10 ou 11, dans lequel l'unité de commande (90) est intégrée dans la poignée (30) du dispositif médical (10) invasif.

13. Agencement de dispositif médical invasif selon la revendication 12, comprenant en outre une batterie (35) couplée à l'au moins une source de courant (60).

14. Agencement de dispositif médical invasif selon l'une quelconque des revendications 10 à 13, dans lequel l'unité de commande (90) comprend en outre un module de communication (37) couplé au microcontrôleur (50), et dans lequel le microcontrôleur est en outre conçu pour commander l'au moins une source de courant (60) pour fournir un courant à la connexion de source de courant (61), laquelle est basé sur un signal de direction (145) pour diriger la pointe (21) flexible orientable reçu d'un système d'imagerie (100) à travers le module de communication.

15. Système d'imagerie (100) comprenant :
l'agencement de dispositif médical invasif selon la revendication 14 ;
un générateur d'image comprenant un générateur de radiateur électromagnétique (120) et un collecteur de rayonnement électromagnétique (130) pour générer une image d'une partie du corps d'un patient (1), ladite partie comprenant au moins la pointe (21) flexible orientable du dispositif médical (10) invasif ;
un processeur d'image (110) couplé au collecteur de rayonnement électromagnétique et conçu :
pour traiter ladite image pour déterminer une orientation réelle de la pointe flexible orientable dans la partie du corps du patient ;
pour déterminer une différence entre ladite orientation réelle et une orientation souhaitée de la pointe flexible orientable ;
pour générer un signal de direction (145) pour la pointe flexible orientable sur la base de ladite différence ; et
pour fournir le signal de direction au module de communication (37) de l'agencement de dispositif médical invasif.
